# EUROPEAN PATENT APPLICATION

(11) **EP 2 438 915 A1**
(43) Date of publication of application: **11.04.2012**
(21) Application number: 11188475.5
(22) Date of filing: 20.07.2007
(51) Int. Cl.: A61K 31/35, A61P 9/08

(54) **Improvement of arginase levels/activity**

(30) Priority: 21.07.2006 US 832684 P
(62) Divisional of application: 07836174.8
(71) Applicant: Mars Incorporated, McLean, VA 22101-3883 (US); Regents of the University of California, Oakland, CA 94607-5200 (US)
(72) Inventor: Schmitz, Harold, Bethesda, MD Maryland 20814 (US); Kwik-Uribe, Catherine, L, Stroudsburg, PA Pennsylvania 18360 (US); Schroeter, Hagen, Davis, CA California 95616 (US)
(74) Representative: Keen, Celia Mary

(57) **Abstract**

This invention relates to compositions, and methods of use thereof, for treating conditions associated with elevated arginase levels and/or activity comprising administering to a subject in need thereof, certain polyphenols such as flavanols, procyanidins, or pharmaceutically acceptable salts or derivatives thereof.

## Description

### FIELD OF THE INVENTION

This invention relates to compositions comprising certain polyphenolic compounds and methods of treating conditions associated with elevated arginase levels and/or arginase activity comprising administering to a subject in need thereof certain polyphenolic compounds described herein.

### BACKGROUND OF THE INVENTION

Arginase is an enzyme which catalyzes the hydrolysis of arginine to ornithine and urea. Ornithine is the biosynthetic precursor of the amino acid proline, which promotes collagen and tissue remodeling, and of polyamines, which play a role in cell proliferation. Arginine is also a substrate for nitric oxide synthase (NOS) which catalyzes the synthesis of nitric oxide (NO) and citrulline from arginine. Thus, arginase competes with NOS for a common substrate.

At least two mammalian isoenzymes of arginase are known to exist (types I and II). Arginase Type I is localized in the cytosol and is predominantly in the liver, but is also expressed in many other cell type, while Type II arginase is mitochondrial and is expressed ubiquitously at low levels in extra-hepatic tissues. Both arginase types I and II can be induced by a variety of cytokines and other agents.

Recently, elevated arginase levels/activity has been shown to be associated with several human pathologies. Therefore, there is a need in the art for methods of modulating/reducing arginase levels/activity.

Applicants have now discovered that the compounds recited herein are effective in reducing gene expression of arginase (*i.e.*, transcriptional level) and/or in reducing arginase activity (*i.e.*, post-transcriptional level). Therefore, compounds recited herein are useful in treating conditions associated with elevated arginase levels/activity.

### SUMMARY OF THE INVENTION

The invention relates to compositions, products and methods of treating conditions associated with elevated arginase levels and/or arginase activity, comprising administering to a subject in need thereof certain polyphenolic compounds described herein.

In one aspect, the invention relates to a composition, such as a food (including pet food), a food additive, a dietary supplement, or a pharmaceutical comprising the compound of the invention. Packaged products containing the above-mentioned compositions and a label and/or instructions for use as described herein, *e.g.*, to reduce arginase levels and/or activity are within the scope of the invention.

In another aspect, the invention relates to a method of treating conditions associated with elevated arginase levels and/or activity comprising administering to a subject in need thereof an effective amount of the compounds of the invention.

In another aspect, the invention relates to a method of reducing arginase levels and/or activity comprising administering to a subject in need thereof an effective amount of the compounds of the invention.

### BRIEF DESCRIPTION OF THE DRAWINGS

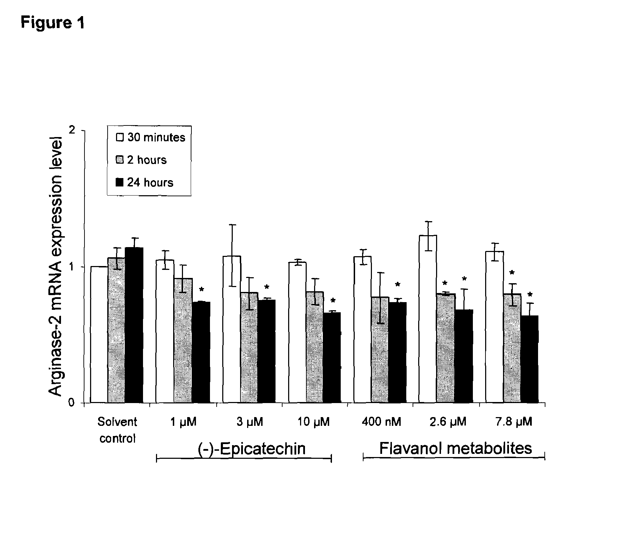

Figure 1: represents Real Time PCR analysis showing that flavanols decrease arginase-2 mRNA-expression in human endothelial cells. Bars represent the mean value of three individual experiments ± SD. For each experiment triplicates were performed. *p<0.05 vs. solvent control.

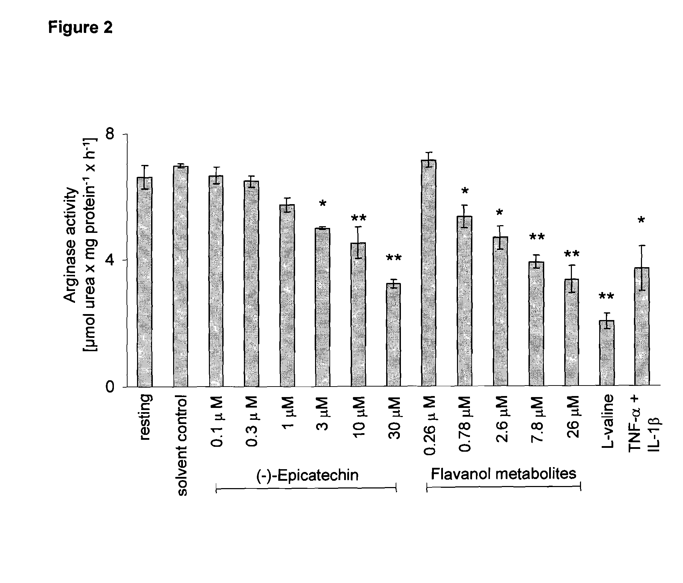

Figure 2: shows that flavanol metabolites and (-)-epicatechin diminish arginase-2 activity in human endothelial cells. Supplementation with the arginase inhibitor L-valine [10 mM] or TNF-α + IL-1β (each 500 U/ml), which decrease arginase expression, were used as positive controls. Bars represent the mean value of four individual experiments ± SD; *p<0.05 vs. solvent control, **p<0.01 vs. solvent control.

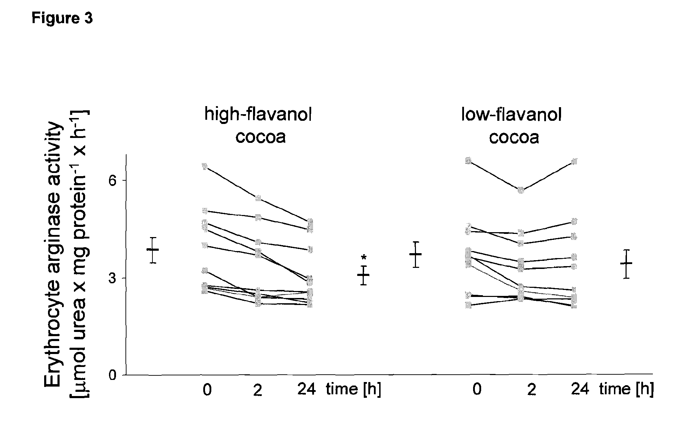

Figure 3: shows that high-flavanol cocoa lowers arginase activity in erythrocytes in healthy humans(n=10); time points before, 2 and 24 hours after ingestion of a 200 ml cocoa drink high in flavanols or the matched low-flavanol control are represented. Black lines represent mean values SE after 0 and 24 hours, grey squares individual values. *p<0.05

### DETAILED DESCRIPTION

All patents, patent applications and references cited in this application are hereby incorporated herein by reference. In case of any inconsistency, the present disclosure governs.

The invention relates to compositions, products and methods for treating conditions associated with elevated arginase levels and/or activity comprising administering to a subject in need thereof certain polyphenolic compounds described herein. The compounds for use in the present invention include certain flavanols (flavan-3-ols), procyanidins, or pharmaceutically acceptable salts or derivatives thereof. Such compounds, when of natural origin, may be included in the composition in the form of a cocoa component, for example cocoa nibs or fragments thereof, chocolate liquor, partially and fully-defatted cocoa solids, cocoa extract or fraction thereof.

As used herein, the term "flavanol" or "flavan-3-ol" refers to a monomer of the following formula:

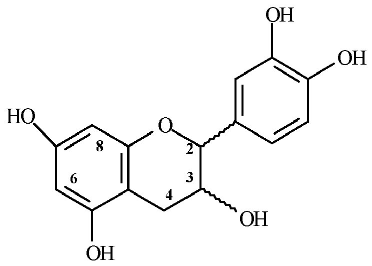

The term "procyanidin" refers to an oligomeric compound composed of monomeric units of the formula shown above.

The term "cocoa component" refers to a component derived from cocoa bean, *e.g.*, cocoa nibs and fragments thereof, chocolate liquor, partially and fully-defatted cocoa solids (*e.g.*, cake or powder), flavanol and/or procyanidin-containing cocoa extract or fraction thereof.

In certain embodiments, the present invention relates to a flavanol (*e.g.*, (-)-epicatechin and (+)-catechin), and a composition comprising an effective amount of the flavanol (*e.g.*, (-)-epicatechin and (+)-catechin), or a pharmaceutically acceptable salt or derivative thereof (including oxidation products, esters, methylated derivatives and glucuronidated derivatives, wherein (in certain embodiments) the flavanol derivative is not a gallated derivative). The derivatives may be prepared as described below.

In other embodiments, the present invention relates to a compound, and a composition comprising an effective amount of the compound, having the following formula Aₙ, or a pharmaceutically acceptable salt or derivative thereof (including oxidation products, esters, methylated derivatives and glucuronidated derivatives): 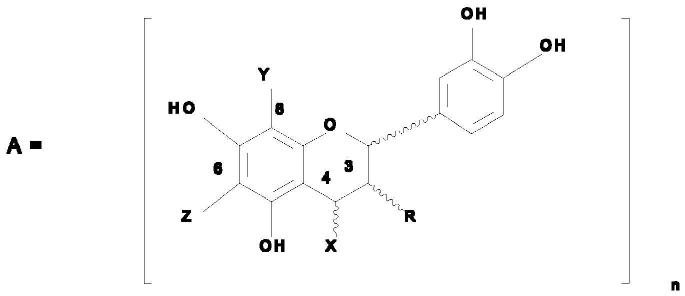 wherein
n is an integer from 2 to 18;
R and X each have either α or β stereochemistry;
R is OH or O-sugar;
the substituents of C-4, C-6 and C-8 are X, Z and Y, respectively, and bonding of monomeric units occurs at C-4, C-6 or C-8;
when any C-4, C-6 or C-8 is not bonded to another monomeric unit, X, Y and Z independently are hydrogen or a sugar; and
the sugar is optionally substituted with a phenolic moiety at any position, for instance, via an ester bond.

Monomeric units in the formula Aₙ may be bonded via 4α→6; 4β→6; 4α→8; and/or 4β→8 linkages. The sugar is preferably a monosaccharide or a di-saccharide. The sugar may be selected from the group consisting of glucose, galactose, rhamnose, xylose, and arabinose. The phenolic moiety may be selected from the group consisting of caffeic, cinnamic, coumaric, ferulic, gallic, hydroxybenzoic and sinapic acids. Procyanidin derivatives may include esters such as the gallate esters; compounds derivatized with a saccharide moiety such as mono- or di-saccharide moiety (*e.g.*, β-D-glucose), glucuronidated and methylated derivatives, and oxidation products. In some embodiments, ester derivatives are other than esters with gallic acid. Oxidation products may be prepared as disclosed in U.S. Pat. No. 5,554,645, the relevant portions of which are incorporated herein by reference. Esters, for example esters with gallic acid, may be prepared using known esterification reactions, and for example as described in US Pat. No. 6,420,572, the disclosure of which is hereby incorporated herein by reference. Methylated derivatives, such as 3'O-methyl-, 4'O-methyl-, and 3'O, 4'O-dimethyl- derivatives may be prepared, for example, as described in Cren-Olive et al., 2002, J. Chem. Soc. Perkin Trans. 1, 821-830, and Donovan et al., Journal of Chromatography B, 726 (1999) 277-283, the disclosures of which are hereby incorporated herein by reference. Glucuronidated products may be prepared as described in Yu et al, "A novel and effective procedure for the preparation of glucuronides," Organic Letters, 2(16) (2000) 2539-41, and as in Spencer et al, "Contrasting influences of glucuronidation and O-methylation of epicatechin on hydrogen peroxide-induced cell death in neurons and fibroblasts," Free Radical Biology and Medicine 31(9) (2001) 1139-46, hereby incorporated herein by reference. Glucuronidation may take place at the 7, 5 and/or 3' position(s). Examples of glucuronidated products include 4'-*O*-methyl- epicatechin-*O*-β -D-glucuronide (*e.g.*, 4'-*O*-methyl- epicatechin-7-*O*-β -D-glucuronide), 3'-*O*-methyl- epicatechin-*O*-β -D-glucuronide (*e.g.*, 3'-*O*-methyl- epicatechin-5/7-*O*-β -D- glucuronides), and epicatechin-*O*-β -D-glucuronide (*e.g.*, epicatechin-7-*O*-β -D-glucuronide). It should be noted that this disclosure applies to all formulas recited herein.

In another embodiment, the invention relates to a compound, and the composition comprising an effective amount the compound having the formula Aₙ, or a pharmaceutically acceptable salt or derivative thereof (including oxidation products, esters, methylated derivatives and glucuronidated derivatives), 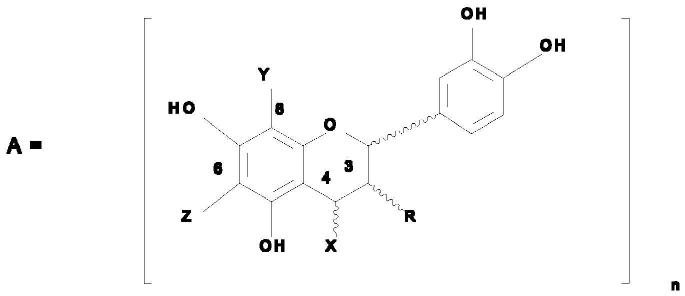 wherein
n is an integer from 2 to 18;
R and X each have either α or β stereochemistry;
R is OH;
the substituents of C-4, C-6 and C-8 are X, Z and Y, respectively, and bonding of monomeric units occurs at C-4, C-6 and C-8; and
when any C-4, C-6 or C-8 is not bonded to another monomeric unit, X, Y and Z are hydrogen.

Examples of the compounds useful for the products and in the methods of the invention include the compounds of the formula Aₙ described herein wherein the integer n is 3 to 18; 2 to 12; 3 to 12; 2 to 5; 4 to 12; 5 to 12; 4 to 10; or 5 to 10. In some embodiments, the integer n is 2 to 4, for example 2 or 3. This disclosure applies to any compound of formula Aₙ herein.

### Methods of Use

The invention relates to methods of treating a condition associated with elevated arginase levels and/or activity in a subject in need thereof.

As used herein, "treatment" or "treating" refers to obtaining a desired pharmacologic effect. The effect may be prophylactic in terms of completely or partially preventing a condition/disease/disorder or a symptom thereof, preventing the disease or a symptom of a disease from occurring in a subject which may be predisposed to the disease but has not yet been diagnosed as having it (*e.g.*, including diseases that may be associated with or caused by a primary disease), and/or may be therapeutic in terms of a slowing down the progression of the condition/disease/disorder (*i.e.*, arresting its development, causing regression of the disease), in prolonging survival, reducing the risk of death, reducing the risk of adverse effects attributable to the disease, and/or inducing a measurable reduction in morbidity associated with the condition/disease/disorder.

As used herein, "treating a condition associated with elevated arginase levels and/or arginase activity," refers to treatment of a condition/disorder/disease associated with an increase in arginase levels and/or arginase activity in a mammal, particularly in a human or a veterinary animal. Examples of such conditions/diseases/disorders are: Hemolytic diseases (*e.g.*, sickle cell anemia, thalassemias, hemolytic disease-associated endothelial dysfunction, hemolytic disease-associated pulmonary hypertension); Inflammation (*e.g.*, impaired wound healing, post-surgical trauma, chronic venous leg ulcer, chronic venous insufficiency, periodontitis, arthritis); Immune disorders/diseases (*e.g.*, Wegener's granulomatosis, arthritis, post-surgical immune dysfunction *e.g.*, in cancer patients); Respiratory/Pulmonary (*e.g.*, asthma, and asthma/respiratory pathology-associated airway hyperreactivity, cystic fibrosis, airway wall thickening and airway remodeling); Vascular/Cardiovascular/Cardiopulmonary (*e.g.*, essential/primary hypertension associated with elevated arginase, pulmonary hypertension *e.g.*, pulmonary arterial hypertension, endothelial dysfunction associated with elevated arginase, angina pectoris, aging vasculature including age-related myocardial dysfunction and heart failure); Microcirculatory dysfunctions (*e.g.*, ischemia-reperfusion injury *e.g.*, in cardiac microvasculature, coronary arteriolar dysfunction, sepsis); Renal dysfunction (*e.g.*, glomerulonephritis, renal insufficiency *e.g.*, in subjects prone to heightened vascular resistance and/or elevated blood pressure); Diabetes-associated conditions (*e.g.*, microcirculatory dysfunction in diabetics *e.g.* in diabetic neuropathy, diabetic nephropathy/renal dysfunction, diabetic foot ulcer including callus formation around ulcer, impaired wound healing in diabetics, microcirculation impairment in peripheral vascular disease in diabetics, diabetes-associated sexual dysfunction); Cancer (*e.g.*, breast, colorectal, prostate); Dermatological (*e.g.*, psoriasis); Neurological (*e.g.*, depression); Sexual Dysfunction (*e.g.*, erectile dysfunction, Peyronie's-like disease of penis, female sexual dysfunction).

The invention relates to treatment of subjects suffering from, or at risk of, the conditions recited above using the compounds described herein.

A person of skill in the art will select the known methods of measuring the levels (*i.e.*, mRNA expression, enzyme (protein) levels)/activity (*i.e.*, enzymatic activity) of arginase for example, methods described in the Example.

In certain embodiments, the present invention provides a method of treating a condition associated with elevated arginase levels and/or activity comprising administering to a mammal (*e.g.*, human) or a veterinary animal in need thereof an effective amount of a flavanol such as epicatechin or catechin (*e.g.*, (-)-epicatechin or (+)-catechin), or a pharmaceutically acceptable salt or derivative thereof (including oxidation products, esters, methylated derivatives and glucuronidated derivatives, wherein the flavanol derivative is not a gallated derivative).

The term "veterinary animal" refers to any animal cared for, or attended to by, a veterinarian, and includes companion (pet) animals and livestock animals, for example a cat, a dog and a horse.

In certain embodiments, the invention provides a method of treating a condition associated with elevated arginase levels and/or activity comprising administering to a mammal (*e.g.*, human) or a veterinary animal in need thereof, a composition comprising an effective amount of a compound having the following formula Aₙ, or a pharmaceutically acceptable salt or derivative thereof (including oxidation products, esters, methylated derivatives and glucuronidated derivatives): 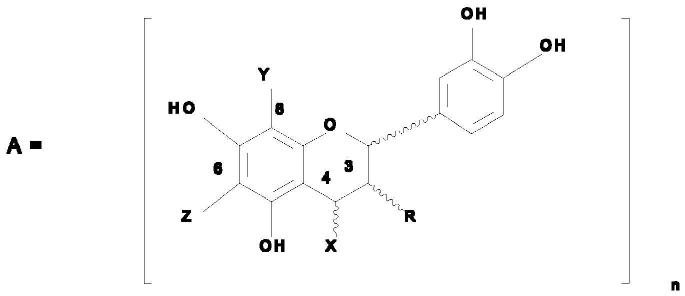 wherein
n is an integer from 2 to 18;
R and X each have either α or β stereochemistry;
R is OH or O-sugar;
the substituents of C-4, C-6 and C-8 are X, Z and Y, respectively, and bonding of monomeric units occurs at C-4, C-6 or C-8;
when any C-4, C-6 or C-8 is not bonded to another monomeric unit, X, Y and Z independently are hydrogen or a sugar; and
the sugar is optionally substituted with a phenolic moiety at any position, for instance, via an ester bond.

For example, the above method may involve use of a compound Aₙ, or a pharmaceutically acceptable salt or derivative thereof (including oxidation products, esters, methylated derivatives and glucuronidated derivatives), wherein R is OH, and when any C-4, C-6 or C-8 is not bonded to another monomeric unit, X, Y and Z are hydrogen. Examples of suitable sugars are as described above. Examples of phenolic moieties are as described above. Examples of derivatives are as described above.

In certain embodiments, the invention provides a method of treating a condition associated with elevated arginase levels and/or activity comprising administering to a mammal (e.g., human) or a veterinary animal in need thereof, a composition comprising an effective amount of a compound having the formula Aₙ, or a pharmaceutically acceptable salt or derivative thereof (including oxidation products, esters, methylated derivatives and 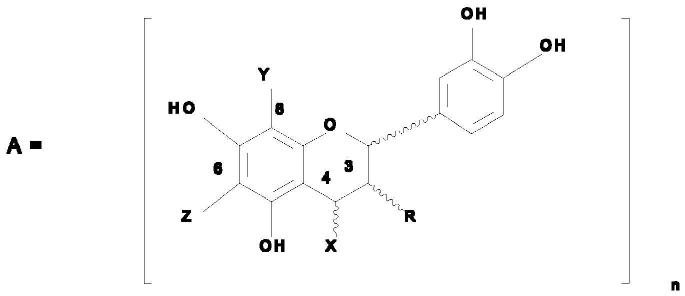 glucuronidated derivatives):
wherein
n is an integer from 2 to 18;
R and X each have either α or β stereochemistry;
R is OH;
the substituents of C-4, C-6 and C-8 are X, Z and Y, respectively, and bonding of monomeric units occurs at C-4, C-6 and C-8; and
when any C-4, C-6 or C-8 is not bonded to another monomeric unit, X, Y and Z are hydrogen.

Examples of the compounds useful for the products and in the methods of the invention include the compounds described herein wherein the integer n is 3 to 18; 2 to 12; 3 to 12; 2 to 5; 4 to 12; 5 to 12; 4 to 10; or 5 to 10. In some embodiments, the integer n is 2 to 4, for example 2 or 3. This disclosure applies to any compound of formula An herein.

Examples of subjects in need of treatment of conditions associated with elevated arginase levels and/or activity will be apparent to those of skill in the art. Examples of such subjects are those suffering from or at risk of: Hemolytic diseases (*e.g.*, sickle cell anemia, thalassemias, hemolytic disease-associated endothelial dysfunction, hemolytic disease-associated pulmonary hypertension); Inflammation (*e.g.*, impaired wound healing, post-surgical trauma, chronic venous leg ulcer, chronic venous insufficiency, periodontitis, arthritis); Immune disorders/diseases (*e.g.*, Wegener's granulomatosis, arthritis, post-surgical immune dysfunction *e.g.*, in cancer patients); Respiratory/Pulmonary (*e.g.*, asthma, and asthma/respiratory pathology-associated airway hyperreactivity, cystic fibrosis, airway wall thickening and airway remodeling); Vascular/Cardiovascular/Cardiopulmonary (*e.g.*, essential/primary hypertension associated with elevated arginase, pulmonary hypertension *e.g.*, pulmonary arterial hypertension, endothelial dysfunction associated with elevated arginase, angina pectoris, aging vasculature including age-related myocardial dysfunction and heart failure); Microcirculatory dysfunctions (*e.g.*, ischemia-reperfusion injury *e.g.*, in cardiac microvasculature, coronary arteriolar dysfunction, sepsis); Renal dysfunction (*e.g.*, glomerulonephritis, renal insufficiency *e.g.*, in subjects prone to heightened vascular resistance and/or elevated blood pressure); Diabetes-associated conditions (*e.g.*, microcirculatory dysfunction in diabetics *e.g.* in diabetic neuropathy, diabetic nephropathy/renal dysfunction, diabetic foot ulcer including callus formation around ulcer, impaired wound healing in diabetics, microcirculation impairment in peripheral vascular disease in diabetics, diabetes-associated sexual dysfunction); Cancer (*e.g.*, breast, colorectal, prostate); Dermatological (*e.g.*, psoriasis); Neurological (*e.g.*, depression); Sexual Dysfunction (*e.g.*, erectile dysfunction, Peyronie's-like disease of penis, female sexual dysfunction).

The present compounds may be administered orally in the form of a cocoa component, for example cocoa nibs or fragments thereof, chocolate liquor, partially and fully-defatted cocoa solids (*e.g.*, cocoa powder), cocoa extract or fraction thereof, or may be added independently of cocoa components. The cocoa component may be prepared such that the content of cocoa polyphenols (CP) (flavanols and/or procyanidins) is preserved.

In some embodiments, the present compounds may be administered in combination with other arginase inhibitors and/or to enhance responsiveness to other arginase inhibitors. Examples of arginase inhibitors include: (S)-(2-Boronoethyl)-L-cysteine (BEC), 2(S)-amino-6-boronohexanoic acid (ABH), N^{G}-Hydroxy-L-arginine (NOHA), L-2-Amino-(4-(2'-hydroxyguanidino) butyric acid (nor-NOHA), and DL-alfa-Difluoromethylornithine (DFMO), and pharmaceutical salts thereof. These additional arginase inhibitors may be administered either topically or orally.

Thus, the following uses are within the scope of the invention. Use of a flavanol, or a pharmaceutically acceptable salt or derivative thereof (including oxidation products, esters, methylated derivatives and glucuronidated derivatives, wherein (in some embodiments) the derivative is not a gallated derivative), as defined above, in the manufacture of a medicament, food, nutraceutical or dietary supplement for treating conditions associated with elevated arginase levels and/or activity or subjects recited above. Use of a compound of formula Aₙ, or a pharmaceutically acceptable salt or derivative thereof (including oxidation products, esters, methylated derivatives and glucuronidated derivatives), as defined herein, in the manufacture of a medicament, food, nutraceutical or dietary supplement for treating conditions associated with elevated arginase levels and/or activity.

The effective amount may be determined by a person of skill in the art using the guidance provided herein and general knowledge in the art, for example by taking into consideration factors such as administered dose, matrix, frequency of dosing, route of administration, *etc.* For example, the effective amount may be such as to achieve a physiologically relevant concentration in the body of a mammal. Such a physiologically relevant concentration may be at least 20 nanomolar (nM), preferably at least about 100 nM, and more preferably at least about 500 nM. In one embodiment, at least about one micromole in the blood of the mammal, such as a human, is achieved. Compounds may be measured in the blood of a subject using methods described for example in Schroeter H. et al., 2006, Proc. Natl. Acad Sci. U.S.A., 103:1024-1029 (see Examples below). The compounds defined herein, may be administered at from about 35 mg/day, 40 mg/day or 50 mg/day (*e.g.*, to about 1000 mg/day), or from about 75 mg/day (*e.g.*, to about 1000 mg/day), or from about 100-150 mg/day (*e.g.,* to about 900 mg/day), or from about 300 mg/day (*e.g.,* to about 500 mg/day). However, amounts higher than exemplified above may be used since the upper end of the amount range is not a limiting factor. The amounts may be measured as described in Adamson, G.E. et al., J. Ag. Food Chem.; 1999; 47 (10) 4184-4188.

The administration may be continued as a regimen, *i.e.*, for an effective period of time, e.g., daily, monthly, bimonthly, biannually, annually, or in some other regimen, as determined by the skilled medical practitioner for such time as is necessary. The administration may be continued for at least a period of time required to reduce arginase levels and/or activity and/or to achieve improvement in a subject recited above. The composition may be administered daily, preferably two or three times a day, for example, morning and evening to maintain the levels of the effective compounds in the body of the mammal. To obtain the most beneficial results, the composition may be administered for at least 7 days, or at least 14 days, or at least 30 days, or at least 45 days, or at least 60 days, or at least 90 days. These regimens may be repeated periodically as needed. The composition may also be beneficial when administered acutely with effects being observable within hours or days, for e.g., with oral administration, or more rapidly with intravenous administration.

### Compositions and Formulations

The compounds of the invention may be administered as a food (including pet food), a food additive, or a dietary supplement, or a pharmaceutical.

As used herein, "food" is a material containing protein, carbohydrate and/or fat, which is used in the body of an organism to sustain growth, repair and vital processes and to furnish energy. Foods may also contain supplementary substances, for example, minerals, vitamins and condiments. See Merriam-Webster's Collegiate Dictionary, 10th Edition, 1993. The term food includes a beverage adapted for human or animal consumption. As used herein a "food additive" is as defined by the FDA in 21 C.F.R. 170.3(e)(1) and includes direct and indirect additives. As used herein, a "dietary supplement" is a product (other than tobacco) that is intended to supplement the diet that bears or contains the one or more of the following dietary ingredients: a vitamin, a mineral, an herb or other botanical, an amino acid, a dietary substance for use by man to supplement the diet by increasing the total daily intake, or a concentrate, metabolite, constituent, extract or combination of these ingredients. As used herein, a "pharmaceutical" is a medicinal drug. See Merriam-Webster's Collegiate Dictionary, 10th Edition, 1993. A pharmaceutical may also be referred to as a medicament. The above compositions may be prepared as is known in the art.

The compositions may contain a carrier, a diluent, or an excipient. Depending on the intended use, the carrier, diluent, or excipient may be chosen to be suitable for human or veterinary use, food, additive, dietary supplement or pharmaceutical use. The composition may optionally contain an additional arginase inhibitor. Also depending on use, a person of skill in the art may select the degree of purity of the compound of the invention. For example, when used to prepare pharmaceutical dosage forms, the compound should be as pure as commercially possible, while when preparing food, additive, or supplement, less pure or mixtures of compounds (*e.g.*, plant extracts) may be used.

The compound of the invention may be "isolated and purified," *i.e.,* it may be separated from compounds with which it naturally occurs (*e.g.*, when the compound is of natural origin), or it may be synthetically prepared, in either case such that the level of contaminating compounds and/or impurities does not significantly contribute to, or detract from, the effectiveness of the compound. For example, an "isolated and purified B2 dimer" is separated from B5 dimer, with which it may occur in nature (*e.g.*, in cocoa bean), to the extent achievable by the available commercially viable purification and separation techniques. Such compounds are particularly suitable for pharmaceutical applications.

The compound may also be less pure, *i.e.*, "substantially pure," *i.e.,* it may possess the highest degree of homogeneity achievable by available purification, separation and/or synthesis technology but need not be separated from the like compounds. As used herein, "the like compounds" are the compounds having the same degree of polymerization. For example, a "substantially pure dimer" refers to a mixture of dimers (*e.g.*, B2 and B5, as it would occur in a cocoa extract fraction). While less suitable for pharmaceutical applications, such "substantially pure" compounds may be utilized for food, food additive and dietary supplement applications.

In some embodiments, the compound of the invention is at least 80% pure, at least 85% pure, at least 90% pure, at least 95% pure, at least 98% pure, or at least 99% pure. Such compounds are particularly suitable for pharmaceutical applications.

Pharmaceuticals containing the inventive compounds, optionally in combination with another arginase inhibitor may be administered orally. As used herein, "oral administration" includes administration by the mouth and includes sublingual and bucal administrations. A person of skill in the art will be able to determine a suitable mode of administration to maximize the delivery of the compounds of the invention. Thus, dosage forms adapted for each type of administration by mouth are within the scope of the invention and include solid, liquid and semi-solid dosage forms, such as tablets, capsules, gelatin capsules (gelcaps), bulk or unit dose powders or granules, emulsions, suspensions, pastes, or jellies. Sustained-release dosage forms are also within the scope of the invention. Suitable pharmaceutically acceptable carriers, diluents, or excipients are generally known in the art and can be determined readily by a person skilled in the art. The tablet, for example, may comprise an effective amount of the compound of the invention and optionally a carrier, such as sorbitol, lactose, cellulose, or dicalcium phosphate.

The foods comprising the compounds described herein and optionally another arginase inhibitor may be adapted for human or veterinary use, and include pet foods. The food may be other than a confectionery, for example, a beverage (*e.g.*, cocoa flavored beverage). A confectionery such as a standard of identity (SOI) and non-SOI chocolate, such as milk, sweet and semi-sweet chocolate including dark chocolate, low fat chocolate and a candy which may be a chocolate covered candy are also within the scope of the invention. Other examples include a baked product (*e.g.*, brownie, baked snack, cookie, biscuit) a condiment, a granola bar, a toffee chew, a meal replacement bar, a spread, a syrup, a powder beverage mix, a cocoa or a chocolate flavored beverage, a pudding, a rice cake, a rice mix, a savory sauce and the like. If desired, the foods may be chocolate or cocoa flavored. Food products may be chocolates and candy bars, such as granola bars, containing nuts, for example, peanuts, walnuts, almonds, and hazelnuts. The food is designed to deliver an effective amount of the compounds described herein.

The compounds for use in the present invention may be of natural origin, for example, derived from a cocoa bean or another natural source known to a person of skill in the art, or prepared synthetically. A person of skill in the art may select natural or synthetic polyphenol based on the use and/or availability or cost.

The compounds may be included in the composition in the form of a cocoa component. For example, the compound(s) may be included in the composition in the form of a cocoa ingredient, for example, chocolate liquor included in chocolate, or may be added independently of cocoa ingredients, for example, as an extract, extract fraction, isolated and purified individual compound, pooled extract fractions or a synthetically prepared compound. The term "cocoa ingredient" refers to a cocoa solids-containing material derived from shell-free cocoa nibs such as chocolate liquor and partially or fully-defatted cocoa solids (*e.g.*, cake or powder). The extraction and purification may be conducted as described in U.S. Pat. Nos. 5,554,645 and 6,670,390 to Romanczyk et al*.,* and U. S. Pat. No. 6,627,232 to Hammerstone et al*.,* each of which is hereby incorporated herein by reference.

Cocoa flavanols and/or procyanidins may be provided in the composition of the invention by cocoa ingredients containing these compounds or by including chocolate, which may be milk, sweet and semi-sweet, and is preferably dark chocolate, and low fat chocolate. The cocoa ingredients may be prepared using traditional cocoa processing procedures but is preferably prepared using the method described in U.S. Pat. No. 6,015,913 to Kealey et al*.* Alternatively, to enhance the level of cocoa polyphenols, chocolate liquor and cocoa solids prepared from cocoa beans having a fermentation factor of 275 or less may be used. These ingredients have cocoa polyphenol content that is higher than can be obtained using traditional cocoa processing methods (*e.g.*, with roasting) and fully fermented beans. The chocolate may be prepared using conventional techniques from the ingredients described above or using an improved process for preserving cocoa polyphenols during chocolate manufacturing as described in the International Appl. No. PCT/US99/05414 published as WO99/45788 and in its U. S. counterpart, U. S. Pat. No. 6,194,020, the relevant portions of which are hereby incorporated herein by reference. A chocolate prepared by at least one of the following non-traditional processes is referred to herein as a "chocolate having a conserved amount of cocoa polyphenols": (i) preparing cocoa ingredients from underfermented or unfermented cocoa beans; (ii) preserving cocoa polyphenol during cocoa ingredient manufacturing process; and (iii) preserving cocoa polyphenol during chocolate manufacturing process. Such non-traditional processes may be used to prepare other cocoa component-containing products (foods *e.g.*, beverages, dietary supplements) designed to contain enhanced levels of flavanols and/or procyanidins.

Synthetic procyanidins may also be used and are prepared by methods known in the art and as described, for example in, U. S. Pat. Nos. 6,420,572; 6,156,912; and 6,864,377, the relevant portions of each of which are hereby incorporated herein by reference.

A daily effective amount of the compound of the invention may be provided in a single serving in case of a food or a single dosage in case of a pharmaceutical or a dietary supplement. For example, a confectionery (*e.g.*, chocolate) may contain at least about 100 mg/serving (*e.g.*, 150-200, 200-400 mg/serving).

The dietary supplement containing the compounds of the invention, and optionally another arginase inhibitor, may be prepared using methods known in the art and may comprise, for example, nutrient such as dicalcium phosphate, magnesium stearate, calcium nitrate, vitamins, and minerals.

Further within the scope of the invention is an article of manufacture such as a packaged product comprising the composition of the invention (*e.g.*, a food, a dietary supplement, a pharmaceutical) and a label indicating the presence of, or an enhanced content of the inventive compounds or directing use of the composition for treating conditions associated with elevated arginase levels and/or activity The packaged product may contain the composition and the instructions for use to reduce arginase levels and/or activity. The label and/or instructions for use may refer to any of the methods of use described in this application.

The invention also relates to a method of manufacturing an article of manufacture comprising any of the compositions described herein, packaging the composition to obtain an article of manufacture and instructing, directing or promoting the use of the composition/article of manufacture for any of the uses described herein. Such instructing, directing or promoting includes advertising.

The invention is further described in the following non-limiting examples.

### EXAMPLES

### Example 1: Cocoa flavanols lower aginase activity and arginase levels in vivo and in vitro.

### Materials and Methods

**Materials:** Chemicals were purchased from Sigma (Deisenhofen, Germany) except when stated otherwise. Metabolite mixture contains a sum of total flavanols of 2.6 µmol/L consisting of (-)-epicatechin (0.1 µmol/l) and catechin (0.4 µmol/l) as well as flavanol metabolites epicatechin-7-β-D-glucuronide (0.25 µmol/l), 4'-O-methyl-epicatechin (0.2 µmol/l), 4'-O-methyl-epicatechin-β-D-glucuronide (1.7 µmol/l). The concentration of each flavanol is equivalent to the plasma concentration two hours after ingestion of high-flavanol containing cocoa beverage (917 mg) as described in Schroeter H. et al., 2006, Proc. Natl. Acad. Sci. U.S.A., 103:1024-1029, hereby incorporated herein by reference.

**Study protocol:** The effect of a cocoa beverage high or low in flavanol (inclusive of oligomers, composition shown below in Table a) was determined in a group of 10 healthy volunteers. Exclusion criteria were smoking, hypertension, diabetes mellitus and terminal renal failure. On two days, arginase activity was measured in erythrocytes, before, 2 and 24 hours after ingestion of either a high-flavanol (902.1 mg) or a low flavanol (36.4 mg) drink as control. The high-flavanol drink contained flavanols and procyanidins as monomers through decamers as shown below in Table b. Individuals were studied in the morning after a 12-hour fasting period. To avoid accumulation effects, investigations were separated by a wash-out phase of more than 6 days.

**Table a: Composition of cocoa beverage**

| | **High CP product** | **Control Product** |
|---|---|---|
| | | |
| Packet size, g | 62 | 62 |
| mg CP | **902.1** | **36.4** |
| Calories | **237.4** | **234.4** |
| Total fat, g | **2.8** | **3.0** |
| Sat. fat, g | **1.5** | **1.7** |
| Cholesterol,mg | **8.8** | **9.7** |
| Sodium, mg | **210.2** | **310.0** |
| Total Carbo., g | **34.3** | **33.0** |
| Dietary Fiber, g | **6.0** | **7.8** |
| Sugars, g | **18.8** | **18.5** |
| Protein, g | **18.8** | **18.8** |
| mg caffeine | **36.5** | **42.4** |
| mg theobromine | **673.0** | **654.8** |
| | | |
| | | |

| **Minerals** | | |
|---|---|---|
| | **High CP product** | **Control Product** |
| | **mg/g** | **mg/g** |
| Sodium, mg | 210.2 | 310.0 |
| Potassium, mg | 1060.2 | 1289.6 |
| Calcium, mg | 487.3 | 482.4 |
| Iron, mg | 3.9 | 5.9 |
| Phosphorus, mg | 560.5 | 530.7 |
| Magnesium, mg | 171.7 | 156.9 |
| Zinc, mg | 3.1 | 3.3 |
| Copper, mg | 0.7 | 0.8 |
| Manganese, mg | 1.2 | 1.1 |

**Table b: CP composition**

| | **HIGH CP** | **Control** |
|---|---|---|
| | % | % |
| monomers | 24.8 | 27 |
| epicatechin | 18.6 | 10.1 |
| catechin | 6.2 | 16.9 |
| dimers | 18.3 | 22.2 |
| trimers | 14.2 | 7.9 |
| tetramers | 12.7 | 5.3 |
| pentamers | 9.8 | 3.9 |
| hexamers | 8.3 | 5.7 |
| heptamers | 3.8 | 4.7 |
| octamers | 3 | 5 |
| nonamers | 4.3 | 11.9 |
| decamers | 0.8 | 6.4 |
| total | 100 | 100 |

**Cell Culture**: Human umbilical vein endothelial cells were purchased from Promo Cell, (Heidelberg, Germany) and were cultured up to 2 passages.

**Quantitative gene expression analysis**: Real-time RT-PCR on the LightCycler® (Roche Diagnostics, Mannheim, Germany) was performed in a total volume of 20 µl containing 2 µl cDNA, 2 µl Fast Start Reaction Mix SYBR Green I, 1.6 µl of 25 mM MgCl2, 2 µl of each primer 5 pmol/µl and 10.4 µl H20. HepG2 cells, human liver cell line were used as positive controls. For negative controls, the same RNA preparations were used with the omission of the reverse transcriptase step. After completion of the cycling process, samples were subjected to a temperature ramp with continuous fluorescence monitoring for melting curve analysis. For each PCR product, apart from primer-dimers, a single narrow peak was obtained by melting curve analysis at the specific melting temperature and only a single band of the predicted size was observed by agarose gel electrophoresis, indicating specific amplification without significant byproducts. Samples were quantified accordingly (LightCycler analysis software, version 3.5) using the house-keeping gene GAPDH as standard.

**Arginase activity:** Erythrocytes from venous blood were separated by differential centrifugation and arginase activity was determined immediately after isolation accordingly (Corraliza I. M. et al., 1994, J. Immunol. Methods 174:231-235). HUVEC cells were lysed and incubated with 10 mM MnCl₂ at 55°C for 10 minutes. Then one volume 0.5 M L-arginine was added and incubated for 60 min at 37°C. The reaction was stopped by adding an 800 µl acid mixture (H₂SO₄:H₃PO₄:H₂O = 1:3:7), and urea was quantified colorimetrically at 540 nm after the addition of 50 µl 9% α isonitrosopropionphenone (ISPF) and heating at 96°C for 45 minutes. The amount of urea produced, after normalization with protein, was used as an index for arginase activity. For the determination of protein concentration in erythrocyte lysates were diluted 1:100. Arginase activity was determined in kidney homogenates prepared from frozen tissue segments according to a modified method (Ensunsa J. L. et al., 2004, Exp. Biol. Med (Maywood) 229:1143-1153) of Brown and Cohen (Brown G.W., Jr., and Cohen, P. P., 1959, J. Biol. Chem. 234:1769-1774).

**GAPDH activity**: Frozen kidney segments were homogenized in 10 volumes of ice-cold buffer (10 mM Tris, pH 7.4, 0.25 M sucrose, 0.5 mM EDTA) and centrifuged at 10,000 x g for 30 minutes at 4°C. GAPDH activity was measured on the resulting supernatants according to Bergmeyer (Bergmeyer H. U., 1972, Methods of enzymatic analysis 2nd edition, Weinheim: VerlagChemie 1; 466-467).

**Animal studies:** Ten male Sprague-Dawley weanling rats were obtained from Charles River Laboratories (Wilmington, MA, USA). Rats were individually housed in suspended stainless-steel cages in a temperature (23°C) and photoperiod (14-10 hr light-dark cycle) controlled room. They were divided into 2 groups (n=5 per group) after a 6 day-adaptation period and given stock diet for 2 days, *ad libitum,* followed by control diet for 4 days, with meal feeding (5 hrs/ day). The animals were then randomly assigned to one of two diet groups and were given restricted access (5 hrs/day during dark cycle: 12 am-5 am) to purified egg white protein-based diet containing 59.5% fructose with either 0% or 4% cocoa powder for 28 days. Both groups were allowed free access to water delivered via a stainless-steel watering system. The detailed composition of the diets is shown in Table 1. The cocoa used for this study was especially high in flavanols and procyanidins and contained 11.0 mg/g epicatechin, 2.8 mg/g catechin, and 43.0 mg/g procyanidins. At the end of the 28-day period, rats were euthanized after a 2 h fast. The animals were anesthetized with carbon dioxide, and kidneys were quickly excised, weighed and stored at -80°C until analysis. Experimental protocols were approved before implementation by the Animal Use and Care Committee at the University of California, Davis, and were administered through the Office of the Campus Veterinarian.

**Statistical analyses:** Values are reported as means SD. Statistical analysis was made by impaired Student's t-test and p <0.05 was considered significant.

### Results

### Treatment with flavanol metabolites decreases arginase-2 mRNA expression in HUVEC

Arginase-2 mRNA expression levels were investigated in human endothelial cells using real-time PCR. Flavanol metabolite composition and concentration used for this *in vitro* study was equivalent to flavanol concentration found in human plasma at two hours after ingestion of a high-flavanol cocoa as described in the method section, amounting to 2.6 µmol/L total flavanols, with 4'-O-methyl-epicatechin-β-D-glucuronide as the major component (Schroeter H. et al., 2006, Proc. Natl. Acad Sci. U.S.A., 103:1024-1029, hereby incorporated herein by reference).

Flavanol metabolite treatment led to lowered arginase-2 mRNA expression after 24 hours of incubation, and in addition, an early response has been observed with 2.6 and 7.8 µmol/L flavanols after 2 hours (Fig. 1). In line with the results obtained with flavanol metabolites, (-)-epicatechin treatment also led to decreased arginase-2 mRNA expression after 24 hours of incubation, whereas there was no short-term effect at 30 minutes or 2 hours.

### (-)-Epicatechin and flavanol metabolites lower arginase activity in HUVEC

Cells were cultured in the presence of epicatechin or a flavanol metabolite mixture, and arginase-2 activity was assayed as described in the methods section. As shown in Figure 2, (-)-epicatechin treatment decreased arginase-2 activity at 48 hours. Arginase activity in control cells was 6.9 ± 0.4 µmol urea x mg protein-1 x h-1, whereas in (-) epicatechin treated cells it was dose-dependently lowered down to 3.3 ± 0.2 µmol urea x mg protein-1x h-1 at 30 µM (-)-epicatechin, comparable to the response in cells challenged with TNF-α and IL-1β (3.7 ± 0.6 µmol urea x mg protein-1 x h-1), a treatment known to cause a decrease in arginase expression (Suchek C.V. et al., 2004, Curr. Mol. Med., 4:763-775). Treatment with a mixture of flavanol metabolites also lowered arginase activity in concentration-dependent manner.

### High-flavanol containing cocoa-based diet lowers arginase activity in vivo

Further evidence on flavanol-mediated effects on arginase activity was provided from an animal experiment. Rats were fed a diet containing 59.5% (w/w) fructose as carbohydrate source with or without 4% (w/w) flavanol-rich cocoa (Table 1), containing (-)-epicatechin and its oligomers. This dietary intervention with high flavanol cocoa resulted in lowered renal arginase activity, whereas GAPDH activity as a control was not affected (Table 2).

| **Table 1: Diet composition (g/kg diet)** | | |
|---|---|---|
| Ingredient | Control | 4%Cocoa |
| | | |
| Egg white¹ | 210 | 210 |
| Corn starch² | 0 | 0 |
| Fructose³ | 595 | 595 |
| Corn oil | 80 | 80 |
| Mineral mix⁴ | 60 | 60 |
| Alphacel⁵ 15 | 40 | 40 |
| High biotin vitamin mix⁶ | 15 | 15 |
| Cocoa power⁷ | 0 | 40 |
| | | |
| 1 Spray dried egg white was obtained from Dyets Inc., (Bethlehem, PA, USA). | | |
| 2Corn starch was obtained from National Starch and Chemical Co., (Bridgewater, NJ, USA). | | |
| 3Fructose was obtained from ICN Biomedicals Inc., (Aurora, OH, USA). | | |
| 4Mineral mix contained the following (g/kg mix): CaCO3, 139.7; CaHPO4, 166.6; K2HPO4. 133.6; NaCl, 21.2; MgSO4, 49.5; FeSO4·7H20, 6.2; ZnCO3, 0.8; MnSO4·H2O, 0.61; CuSO4·5H2O, 0.66; KI, 0.0033; CrK(SO4)2·12H2O, 0.048; Na2SeO3, 0.015; Na2MoO4·2H20, 0.0063, Cerelose 481.06 | | |
| 5Alphacel, nonnutritive bulk, was obtained from INC Biomedicals Inc. (Aurora, OH, USA). | | |
| 6Vitamin mix contained the following (g/kg mix); inositol, 25.0; ascorbic acid, 5.0; calcium pantothenate, 0.67; thiamine hydrochloride, 0.27; pyridoxine hydrochloride, 0.53; nicotininc acid, 1.0; menadione, 0.25; riboflavin, 0.27; ρ-aminobenzoic acid, 0.50; folic acid, 0.067; biotin, 0.26; all-rac-α-tocopherol, 1.20; retinol, 0.047; cholecalciferol 0.0017; vitamin B12 + mannitol, 3.33; choline chloride (70% sol mL/kg), 71.50; cerelose, 887.52 | | |
| 7Cocoa powder was obtained from Mars Inc. (Hackettstown, NJ, USA). It contained 13.8 mg/g monomer including epicatechin 11.0 mg/g epicatechin and 2.8 mg/d catechin, 10.5 mg/g dimmer, 7.7 mg/g trimer, | | |
| 6.7 mg/g tetramer, 5.1 mg/g pentamer, 4.2 mg/g hexamer, 2.3 mg/g heptamer, 2.1 mg/g octamer, 3.2 mg/g nonamer and 1.2 mg/g decamer | | |

| **Table 2: Cocoa powder-containing diet lowers arginase activity in rat kidney** | | |
|---|---|---|
| Enzyme activity [U/mg protein] | Control | 4%Cocoa |
| Arginase | 0.18 ± 0.02 | 0.13 ± 0.02* |
| GAPDH | 1.30 ± 0.10 | 1.25 ± 0.11 |
| | | |
| Rats fed with a casein-based diet for 28 days containing fructose 59.9% (w/w) as carbohydrate source with or without 4% (w/w) cocoa (see Table 1). Data are given as means SD. *p<0.05 | | |

### High-flavanol cocoa consumption lowers arginase activity in erythrocytes in humans

In an *in vivo* study in healthy humans (n=10) using a high-flavanol cocoa drink and a matched low-flavanol control drink, arginase activity was assayed in erythrocytes before, 2 and 24 hours after the ingestion of a 200 mL cocoa drink. Consumption of the high flavanol containing cocoa drink resulted in a slight decrease of arginase activity at 2 hours (mean value: 3.4 ± 0.4 µmol urea x mg protein-1 x h-1; p<0.05) compared to control (3.9 ± 0.4 µmol urea x mg protein-1 x h-1). However, a pronounced decrease in arginase activity was seen at 24 hours (3.0 ± 0.3 µmol urea x mg protein-1 x h-1; p<0.05). Ingestion of a low-flavanol cocoa drink also led to a small decrease of enzyme activity at 2 hours (3.3 ± 0.4 vs. 3.7 ± 0.4 µmol urea x mg protein-1 x h-1; p<0.05), whereas arginase activity was not substantially affected at 24 hours (3.5 ± 0.5 µmol urea x mg protein-1 x h-1; p=n.s.) as compared to controls (Figure 3).

## Claims

1. A composition for use in treating a condition associated with elevated arginase levels and/or arginase activity in a subject in need thereof, wherein the composition comprises an effective amount of at least one compound selected from epicatechin, catechin, a pharmaceutically acceptable salt thereof, and a derivative thereof, wherein the derivative is not a gallated derivative; and a polymeric compound having the formula Aₙ, a pharmaceutically acceptable salt thereof, and a derivative thereof: 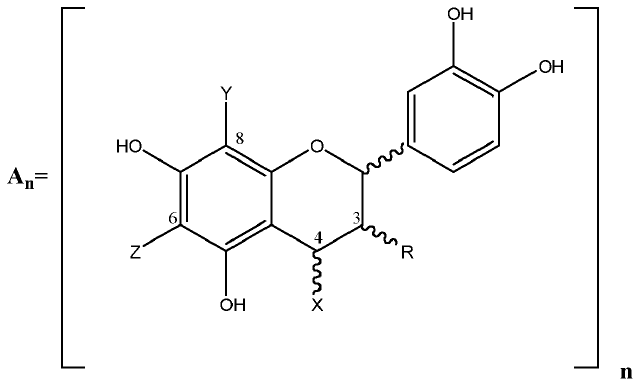 wherein
n is an integer from 2 to 18;
R and X each have either α or β stereochemistry;
R is OH, O-sugar or O-gallate;
the substituents of C-4, C-6 and C-8 are X, Z and Y, respectively, and bonding of monomeric units occurs at C-4, C-6 or C-8;
when any C-4, C-6 or C-8 is not bonded to another monomeric unit, X, Y and Z are hydrogen or a sugar; and
the sugar is optionally substituted with a phenolic moiety.

2. A composition for use according to claim 1, wherein the subject is a human.

3. A composition for use according to claim 2, wherein the subj ect suffers from a condition selected from microcirculatory dysfunction and a diabetes-associated condition.

4. A composition for use according to claim 3, wherein the compound is epicatechin and/or a pharmaceutically acceptable salt thereof.

5. A composition for use according to claim 1, wherein the derivative is a methylated derivative selected from 3'-O-methyl-(+)catechin, 3'-O-methyl-(-)-epicatechin, 4'-O-methyl-(+)-catechin, 4'-O-methyl-(-)-epicatechin, 3'-O-, 4'-O-dimethyl-(+)-catechin, and 3'-O-,4'-O-dimethyl-(-)-epicatechin.

6. A composition for use according to clam 1 wherein the derivative is selected from 4'-O-methyl epicatechin-O-β-D-glucuronide (*e.g.*, 4'-O-methyl-epicatechin-7-O-β -D-glucuronide, 3'-O-methyl- epicatechin-O-β -D- glucuronide (*e.g.*, 3'-O-methyl- epicatechin-5/7-O-β -D- glucuronides), and epicatechin-O-β -D glucuronide (*e.g.*, epicatechin-7-O-β -D-glucuronide).

7. A composition for use according to claim 1, wherein the compound is a polymeric compound of the formula Aₙ wherein n is 2 to 10, R is OH, and X, Y and Z are selected from the group consisting of monomeric unit A and hydrogen, or a pharmaceutically acceptable salt thereof.

8. A composition for use according to claim 7, wherein the adjacent monomeric units bind at positions (4β→6) and/or (4β→8).

9. A composition for use according to claim 1, wherein the composition is a pharmaceutical, a food, a pet food, a food additive, a beverage or a dietary supplement.

10. A composition for use according to claim 9, wherein the food is a confectionery or chocolate.

11. A composition for use according to claim 1, wherein the compound is provided as a cocoa extract, a cocoa extract fraction or a cocoa solid.

12. A composition for use according to claim 11, wherein the cocoa solid comprises a conserved level of epicatechin.

13. A composition for use according to claim 11 or 12, wherein the cocoa solid is a cocoa powder or a non-alkalized cocoa solid.

14. A composition for use in treating a subject suffering from a condition selected from hemolytic disease, pulmonary hypertension, cystic fibrosis, diabetes-associated microcirculatory dysfunction, diabetic foot ulcer, and sexual dysfunction, wherein the composition comprises an effective amount of at least one compound selected from epicatechin, catechin, a pharmaceutically acceptable salt, and a derivative thereof, wherein the derivative is not a gallated derivative; and a polymeric compound having the formula Aₙ, a pharmaceutically acceptable salt thereof, and a derivative thereof: 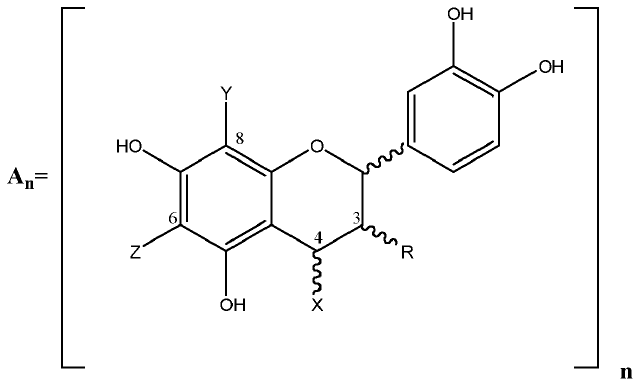 wherein
n is an integer from 2 to 18;
R and X each have either α or β stereochemistry;
R is OH, O-sugar or O-gallate;
the substituents of C-4, C-6 and C-8 are X, Z and Y, respectively, and bonding of monomeric units occurs at C-4, C-6 or C-8;
when any C-4, C-6 or C-8 is not bonded to another monomeric unit, X, Y and Z are hydrogen or a sugar; and
the sugar is optionally substituted with a phenolic moiety at any position.

15. A composition for use according to claim 14, wherein the condition is microcirculatory dysfunction in a diabetic subject.
